# EUROPEAN PATENT SPECIFICATION

(11) **EP 4 433 153 B1**
(45) Date of publication and mention of the grant of the patent: **14.10.2026**
(21) Application number: 22834798.5
(22) Date of filing: 18.11.2022
(51) Int. Cl.: A61N 1/362, A61N 1/37, A61N 1/372, A61N 1/375, A61N 1/365

(54) **IMPLANTABLE PACEMAKER WITH AUTOMATIC IMPLANT DETECTION AND SYSTEM INTEGRITY DETERMINATION**
IMPLANTIERBARER HERZSCHRITTMACHER MIT AUTOMATISCHER IMPLANTATERKENNUNG UND SYSTEMINTEGRITÄTSBESTIMMUNG
STIMULATEUR CARDIAQUE IMPLANTABLE À DÉTECTION AUTOMATIQUE D'IMPLANT ET DÉTERMINATION D'INTÉGRITÉ DE SYSTÈME

(30) Priority: 18.11.2021 US 202163264252 P; 17.11.2022 US 202218056472
(43) Date of publication of application: 25.09.2024
(73) Proprietor: Medtronic, Inc., Minneapolis, Minnesota 55432 (US)
(72) Inventor: STROEBEL, John C., Mounds View, Minnesota 55112 (US); LYBARGER, Maureen E., Mounds View, Minnesota 55112 (US); TEKMEN, Mohac, Mounds View, Minnesota 55112 (US); HERR, Greggory R., Mounds View, Minnesota 55112 (US); GOLNITZ, John D., Mounds View, Minnesota 55112 (US); CATTIN, Kristen J., Mounds View, Minnesota 55112 (US); SCHILLING, Eric A., Mounds View, Minnesota 55112 (US); WILLIAMS, Eric R., Mounds View, Minnesota 55112 (US); WHITMAN, Teresa A., Mounds View, Minnesota 55112 (US); HOLM, Mikayle A., Mounds View, Minnesota 55112 (US); GALARNEAU, Michelle M., Mounds View, Minnesota 55112 (US); TREML, Tara M., Mounds View, Minnesota 55112 (US); PRUSENER, Derek W., Mounds View, Minnesota 55112 (US)
(74) Representative: Zimmermann & Partner Patentanwälte mbB
(86) International application number: PCT/US2022/050446
(87) International publication number: WO 2023/091691

(56) References cited:
- WO-A1-2013/049265
- US-A1- 2012 109 235
- US-A1- 2018 161 572
- US-A1- 2020 094 061
- US-B2- 8 463 382

## Description

### BACKGROUND

Implantable medical devices (IMDs), such as cardiac pacemakers, are often configured to be connected to leads. The leads include electrical conductors that extend through a lead body from a connector assembly provided at a proximal lead end to one or more electrodes located at the distal lead end or elsewhere along the length of the lead body. The conductors connect stimulation and/or sensing circuitry within the IMD housing to respective electrodes or other sensors on the lead.

Therapeutic electrical signals provided by the leads connected to the IMD may include pulses or shocks for pacing, cardioversion, or defibrillation. In some cases, an IMD senses intrinsic depolarizations of the heart, and controls delivery of therapeutic signals to the heart based on the sensed depolarizations. An IMD may also be used to conduct temporary cardiac pacing on a temporary basis (for example, up to about 90 days). Temporary pacing may be prescribed to patients who may have temporary conduction disturbances, such as following the end of an operative procedure, or as a bridge between permanent implants in cases of device or system infection. In some examples, conduction disturbances treatable using a temporary IMD may be the result of transcatheter aortic valve replacement (TAVR), or may be caused by alcohol septal ablation or Lyme carditis.

The implantation of an IMD, such as a temporary or permanent pacemaker, has typically required one or more custom support instruments such as, for example, a device programmer, to perform a number of confirmation checks on the IMD at the time of implant. These confirmation checks help to ensure that the IMD and leads are properly implanted and operational post implant procedure, and can be important to ensure that the system provides prescribed therapy to a patient beginning at the time of implant.

US 8 463 382 B2 relates to an implantable lead functional status monitor and method.

WO 2013/049265 A1 relates to an IMD stability monitor.

### SUMMARY

The invention is defined by the independent claims 1 and 15. When a temporary or permanent IMD is implanted, in some cases the practitioner performing the implant procedure lacks experience, or performs the implant procedure infrequently. In other cases, the implant procedure may be performed at a location or under emergency conditions where the practitioner performing the implant procedure may not have access to custom device programming instruments. In such situations the practitioner may desire rapid feedback on whether the implantation procedure was performed successfully. In some examples, the most readily available feedback on proper implantation is a surface electrocardiogram (ECG), which, in the absence of a sophisticated external programmer, can be monitored by the clinician performing the implant procedure.

In general, the present disclosure is directed to techniques in which, after leads are attached to the IMD, the IMD triggers performance of diagnostic self-tests and provides rapid feedback to the practitioner to support the implant procedure. In some examples, following lead connection, the temporary or permanent IMD performs at least some of the following diagnostic tests: detection of qualification of connection to a pacing electrode(s), determination that the IMD is able to adequately sense intrinsic cardiac activity of the patient, determination that pacing operations generated by a signal generator or an implantable pulse generator (IPG) in the IMD are acceptable for implant, e.g., that pacing pulses capture the heart, and detection of proper lead fixation via assessment of current of injury (COI) parameters.

In some examples, the IMD performs periodic lead impedance monitoring to detect connection to electrodes, integrity of the lead, and/or connection of the electrodes to the heart. In some examples, detection of lead impedance in a valid range triggers other diagnostic tests such as monitoring a patient electrogram (EGM) to allow measurement of amplitudes alone or in combination with calculation of COI parameters. If the EGM amplitude and COI parameters are satisfied, the IMD may perform other test or measurements. In some examples, the IMD may lower a pacing rate, if necessary, to allow intrinsic conduction to determine EGM amplitudes, e.g., to confirm R-wave detection. In some examples, after EGM sensing has been confirmed, or it is determined that intrinsic conduction does not occur even when the pacing rate is lowered to threshold, e.g., indicating pacer dependency, IMD may perform capture tests to determine the pacing capture threshold (PCT), e.g., to determine whether the PCT is within an acceptable range for operation of the IMD.

In some examples, if the IMD does not itself make capture determinations, a clinician may observe the rhythm of the patient on an ECG monitor, and determine adequate R-wave sensing and device capture based on these observations. In some examples, the IMD confirms device capture by pacing the heart and detecting a signal confirming device capture. In some examples, IMD repeats the qualification test cycle for a predetermined period of time (for example, about 30 minutes) to provide continued monitoring of pacing and sensing effectiveness until (and in some cases after) the implantation procedure is complete (e.g., until or after skin closure). The IMD may provide confirmation of pacing and sensing effectiveness to a clinician via a confirmation signal, as described herein.

In some examples, the IMD performs these self-test algorithms without any external instrument or programming device, other than an optional ECG monitor that can display heart rate with some accuracy, and as such the method of the present disclosure can simplify the IMD implantation procedure and evaluation of device capture. In this manner, the techniques of this disclosure may improve the performance of the IMD during an implantation procedure or other performance evaluation, particularly in cases where custom support instruments may not be available.

In one aspect, the present disclosure is directed to an implantable medical device (IMD) configured to be coupled to at least one implantable medical lead, wherein the IMD comprises: sensing circuitry configured to sense an electrogram (EMG) signal of a patient via at least one electrode of the implantable medical lead; impedance measurement circuitry to measure impedance via the implantable medical lead; and a processor. The processor is configured, in response to coupling of the IMD to the at least one implantable medical lead, to initiate a device test sequence comprising a plurality of qualification tests over an evaluation period in which the processor: (1) controls the impedance measurement circuitry to measure an impedance for at least one electrical path that includes the at least one electrode to determine a connection status of the IMD to the at least one electrode; and (2) compares EGM (electrogram) amplitudes of the patient over an EGM test period against a predetermined threshold.

In another aspect, the present disclosure is directed to a non-claimed method comprising
detecting, by an implantable medical device (IMD), attachment to the IMD of at least one implantable medical lead, wherein the at least one implantable medical lead comprises at least one electrode, and triggering by the IMD, based on the detecting of the attachment to the IMD of the at least one medical lead, a device test sequence in which the IMD performs the following qualification tests over an evaluation period: (1) detecting an impedance for at least one electrical path that includes the at least one electrode to determine a connection status of the IMD to the at least one electrode; and (2) comparing EGM (electrogram) amplitudes of the patient over an EGM test period against a predetermined threshold.

In another aspect, the present disclosure is directed to a computer-readable medium comprising instructions that cause a processor to: following connection of the implantable medical device (IMD) to at least one lead, the lead including an electrode, controlling the IMD to automatically initiate, without input from an external programming device, a device test sequence in which the IMD performs the following qualification tests over an evaluation period: (1) detecting an impedance for at least one electrical path that includes the at least one electrode to determine a connection status of the IMD to the at least one electrode; and (2) comparing, over an EGM test period, cardiac sensed event amplitudes in an electrogram of the patient over an EGM test period against a predetermined threshold.

The details of one or more examples are set forth in the accompanying drawings and the description below. Other features, objects, and advantages of the disclosure will be apparent from the description and drawings, and from the claims.

### BRIEF DESCRIPTION OF THE DRAWINGS

FIG. 1 is a conceptual drawing illustrating an example system that includes a temporary or permanent implantable medical device (IMD) coupled to implantable medical leads.
FIG. 2 is a functional block diagram illustrating an example configuration of the IMD of FIG. 1.
FIGS. 3-6 and 7A-7C are flow diagrams of example operations according device test sequences that may be performed, at least in part, by an IMD according to the present disclosure.
FIG. 8 is a conceptual diagram illustrating a configuration of another example IMD.

Like symbols in the drawings indicate like elements.

### DETAILED DESCRIPTION

In general, in this application an IMD is configured to perform a number of diagnostic tests following initial implantation in an automated fashion. In some examples, these diagnostic tests are performed by the IMD without input from an external programmer or other monitoring device. In some examples, the diagnostic tests performed by the IMD include periodic lead impedance monitoring to detect connection to electrodes, which in some examples is triggered by attachment of leads to the IMD. In some examples, detection of lead impedance in a valid range triggers measurement by the IMD of EGM amplitudes, and may include lowering the pacing rate generated by the IMD, if necessary, to uncover intrinsic signals, e.g., R-waves, in the EGM signals. In some examples, detection of lead impedance in a valid range can trigger measurement by the IMD of COI parameters. In some examples, after sensing of R-waves or another indication of adequate sensed EGM amplitudes, or it is determined that the patient does not have intrinsic conduction detectable at a lower escape interval, the IMD performs testing of pacing capture.

In some examples, the IMD presents a confirmation signal to the user based on successful completion of the test sequence, e.g., after determining an adequate pacing capture threshold. In some examples, the confirmation is observable on an ECG by a clinician as a fixed pacing rate, or pacing pattern such as, for example, alternating rates and/or alternating pacing outputs, over a predetermined time period. The confirmation cycle may repeat for a period of time (for example, about 30 minutes) to provide continued monitoring of pacing and sensing effectiveness as (and in some cases after) the implantation procedure is completed. The IMD may provide confirmation of pacing and sensing effectiveness to a clinician via a confirmation signal, as described herein.

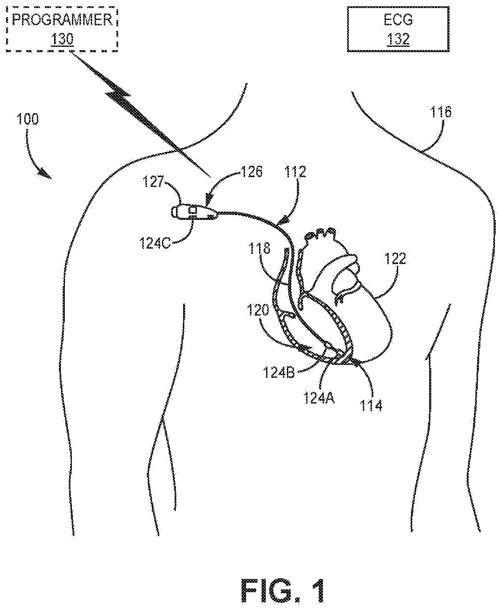

FIG. 1 is a conceptual diagram illustrating a portion of an example implantable medical device system 100 in accordance with one or more aspects of this disclosure. In the example of FIG. 1, implantable medical device system 100 includes one or more implantable medical leads 112 and an implantable medical device (IMD) 126. Implantable medical lead 112 includes an elongated lead body 118 with a distal portion 120. Distal portion 120 of implantable medical lead 112 is positioned at a target site 114 within a heart 122 of a patient 116. Distal portion 120 may include one or more electrodes. Target site 114 may be located at a wall of a ventricle of heart 122. In various examples, the lead 112 may be a unipolar, a bipolar, or a multipolar lead.

A clinician may maneuver distal portion 120 through the vasculature of patient 116 to position distal portion 120 at or near target site 114. For example, the clinician may guide distal portion 120 through the superior vena cava (SVC) to target site 114 on or in a ventricular wall of heart 122, e.g., at the apex of the right ventricle as illustrated in FIG. 1. In some examples, other pathways or techniques may be used to guide distal portion 120 into other target implant sites within the body of patient 116. Other target implant sites may include the ventricular septum, e.g., for delivery of conduction system pacing via one or more of the His bundle, the right bundle branch, or the left bundle branch. Implantable medical device system 100 may include a delivery catheter and/or outer member (not shown), and implantable medical lead 112 may be guided and/or maneuvered within a lumen of the delivery catheter in order to approach target site 114.

Implantable medical lead 112 may include electrodes 124A and 124B configured to be positioned on, within, or near cardiac tissue at or near target site 114, and a housing 127 of IMD 126 may include a housing electrode 124C. Electrodes 124A-124C may collectively be referred to "electrodes 124", and the number and locations of electrodes 124 shown in FIG. 1 are merely examples. In some examples, electrodes 124 are configured to function as electrodes to, for example, sense EGM signals of heart 122 and provide pacing to heart 122.

Electrodes 124 may be electrically connected to conductors (not shown) extending through lead body 118. In some examples, the conductors are electrically connected to therapy delivery circuitry of IMD 126, with the therapy delivery circuitry configured to provide electrical signals through the conductor to electrodes 124. Electrodes 124 may conduct the electrical signals to the target tissue of heart 122, causing the cardiac muscle, e.g., of the ventricles, to depolarize and, in turn, contract at a regular interval. Electrodes 124 may also be connected to sensing circuitry of IMD 126 via the conductors, and the sensing circuitry may sense activity of heart 122 via electrodes 124. Electrodes 124 may have various shapes such as tines, helices, screws, rings, and so on. Again, although a bipolar configuration of lead 112 including two electrodes 124 is illustrated in FIG. 1, in other examples IMD 126 may be coupled to leads including different numbers of electrodes, such as one electrode, three electrodes, or four electrodes.

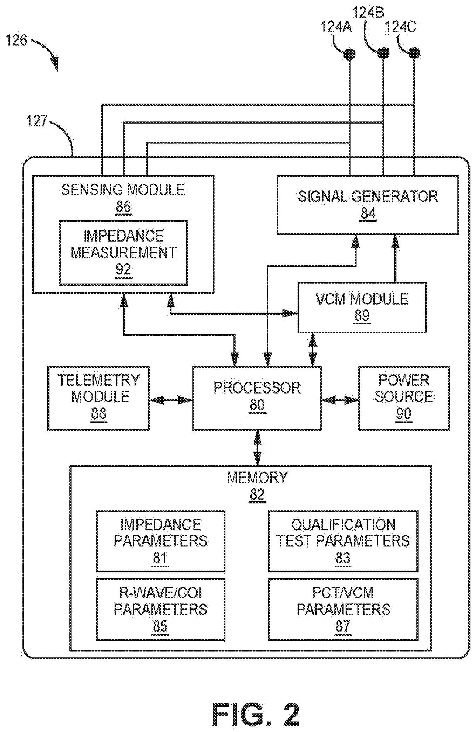

In some examples, one or more housing electrodes 124C may be formed integrally with an outer surface of housing 127 or otherwise coupled to the housing 127. In some examples, housing electrode 124C is defined by an uninsulated portion of an outward facing portion of the housing 127 of the IMD 126. Other divisions between insulated and uninsulated portions of the housing 127 may be employed to define two or more housing electrodes. In some examples, the housing electrode 124C can include substantially all of the housing 127. Any of the electrodes 124A, 124B may be used for unipolar sensing or pacing in combination with the housing electrode 124C. As described in further detail with reference to FIG. 2, the housing 127 may enclose therapy delivery circuitry, referred to as a stimulation signal generator, that generates cardiac pacing pulses, as well as a sensing module including sensing circuitry for monitoring the patient's heart rhythm.

The configuration of the therapy system 100 illustrated in FIG. 1 is merely one example. In other examples, a therapy system may include epicardial leads and/or patch electrodes instead of or in addition to the transvenous lead 112 illustrated in FIG. 1. Further, the IMD 126 need not be implanted within the patient 116. In examples in which the IMD 126 is not implanted in the patient 116, the IMD 12 may deliver therapies to the heart 122 via percutaneous leads that extend through the skin of patient 116 to a variety of positions within or outside of heart 122.

In one or more examples, IMD 126 includes electronic circuitry contained within an enclosure where the circuitry may be configured to deliver cardiac pacing. In the example of FIG. 1, the electronic circuitry within IMD 126 may include therapy delivery circuitry electrically coupled to electrodes 124. The electronic circuitry within IMD 126 may also include sensing circuitry configured to sense electrical activity of heart 122 via electrodes 124. The therapy delivery circuitry may be configured to administer cardiac pacing via electrodes 124, e.g., by delivering pacing pulses in response to expiration of a timer and/or in response to detection of the intrinsic activity (or absence thereof) of the heart.

In some examples, the system 100 includes an optional programmer 130. For example, optional programmer 130 can be a handheld computing device such as a tablet or a phone, a computer workstation, or a networked computing device. The optional programmer 130 can include a user interface that receives input from a clinician, which can include a keypad and a suitable display such as, for example, a touch screen display, or a peripheral pointing device, such as a mouse, via which a user may interact with the user interface. The clinician may also interact with the programmer 130 remotely via a networked computing device.

The clinician, such as a physician, technician, surgeon, electrophysiologist, and the like, may in some cases interact with the programmer 130 to communicate with the IMD 126. For example, the clinician may interact with the programmer 130 to retrieve physiological or diagnostic information from the IMD 126. The clinician may also interact with the programmer 126 to program the IMD 126, e.g., select values for operational parameters of the IMD. In some examples, the programmer 130 may include an optional electrocardiogram (ECG) monitor.

The IMD 126 and the programmer 130 may communicate via wireless communication using any techniques known in the art. Examples of communication techniques may include, for example, low frequency or radiofrequency (RF) telemetry, but other techniques are also contemplated. In some examples, the IMD 126 may signal the programmer 130 to further communicate with and pass information through a network such as those available under the trade designation Medtronic CareLink Network from Medtronic, Inc., of Minneapolis, MN, or some other network linking the patient 116 to a clinician.

In some examples, the system 100 includes an optional electrocardiogram (ECG) monitor 132. In some methods of the present disclosure, if the programmer 130 is not available, the EGC monitor 132 can provide a clinician with feedback regarding the rhythm and electrical activity of the heart following an IMD implantation procedure. In various examples, the ECG monitor can include one or more leads (not shown in FIG. 1), may include surface electrodes attached to the skin of the patient (not shown in FIG. 1), or may be a wireless monitor without leads.

FIG. 2 is a functional block diagram illustrating an example configuration of an example IMD such as the IMD 126 described in FIG. 1. In the illustrated example, the IMD 126 includes a processor 80, a memory 82, a signal generator 84, a sensing module 86, a telemetry module 88, a ventricular capture management (VCM) module 89, and power source 90. The memory 82 includes computer-readable instructions that, when executed by the processor 80, cause the IMD 126 and the processor 80 to perform various functions described herein. The memory 82 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital or analog media.

Processor 80 may include processing circuitry, such as any one or more of a microprocessor, a controller, a digital signal processor (DSP), an application specific integrated circuit (ASIC), a field-programmable gate array (FPGA), or equivalent discrete or analog logic circuitry. In some examples, the processor 80 may include multiple components, such as any combination of one or more microprocessors, one or more controllers, one or more DSPs, one or more ASICs, or one or more FPGAs, as well as other discrete or integrated logic circuitry. The functions attributed to the processor 80 herein may be embodied as software, firmware, hardware or any combination thereof.

Processor 80 controls signal generator 84 to deliver therapy to heart 122 according to a selected one or more of therapy programs, which may be stored in memory 82. For example, processor 80 may control signal generator 84 to deliver electrical pulses with the amplitudes, pulse widths, frequency, or electrode polarities specified by the selected one or more therapy programs. As discussed in more detail below, processor 80 may control the signal generator to pace the heart in a number of different modes including, but not limited to, VOO, VVI, and OVO.

Signal generator 84 is electrically coupled to electrodes 124A, 124B, e.g., via conductors of lead 120, or, in the case of housing electrode 124C, via an electrical conductor disposed within the housing 127 of the IMD 126. Signal generator 84 includes circuitry, such as capacitors, charge pumps, regulators, current mirrors, and switches, configured to generate and deliver electrical signals to heart 122. For example, signal generator 84 may deliver pacing stimulation in the form of electrical pulses via the electrodes 124A-C. Signal generator 84 may include switches, and processor 80 may use the switches to select which of the available electrodes 124 are used to deliver therapy signals. The switches may include a switch array, switch matrix, multiplexer, or any other type of switching device suitable to selectively couple stimulation energy to selected electrodes.

Sensing module 86 monitors signals from at least one of electrodes 124A-C to monitor electrical activity of heart 122. Sensing module 86 may include electrical sensing circuitry such as filters and amplifiers, as well as an analog-to-digital converter. Sensing module 86 may also include switches to select which of the available electrodes are used to sense the heart activity, e.g., to sense a cardiac EGM, depending upon which electrode combination is used in the current sensing configuration. Sensing module 86 may include one or more detection channels, each of which may be coupled to a respective electrode combination and include an amplifier. The detection channels may be used to sense respective cardiac EGMs. Some detection channels may be configured detect cardiac events, such as, for example, R- or P-waves, or COI parameters, and provide indications of the occurrences of such events to the processor 80. In some examples, processor 80 detects cardiac events or determines other parameters discussed herein, e.g., COI parameters, based on a digitally converted EGM signal.

For generation and delivery of pacing pulses to the heart 122, processor 80 may utilize programmable counters to control the basic time intervals associated with VOO, OVO, DDD, VVI, DVI, VDD, AAI, DDI, DDDR, VVIR, DVIR, VDDR, AAIR, DDIR and other modes of single and dual chamber pacing. In the aforementioned pacing modes, "D" may indicate dual chamber, "V" may indicate a ventricle, "I" may indicate inhibited pacing (e.g., no pacing), and "A" may indicate an atrium. The first letter in the pacing mode may indicate the chamber that is paced, the second letter may indicate the chamber that is sensed, and the third letter may indicate the chamber in which the response to sensing is provided. The intervals may include atrial and ventricular pacing escape intervals, refractory periods during which sensed P-waves and R-waves are ineffective to restart timing of the escape intervals. Processor 80 may also define a blanking period, and provide signals to the sensing module 86 to blank one or more channels, e.g., amplifiers, for a period during and after delivery of electrical stimulation to heart 122. The durations of these intervals may be determined by processor 80 in response to stored data in memory 82.

As discussed above, IMD 126 may implement a number of techniques that improve its operation to, for example, verify satisfactory implantation in a way that may be automated and not require programmer 130. To that end, following attachment of a lead to the IMD 126, processor 80 may be configured to control IMD 126 to initiate a device test sequence according to qualification test parameters 83 stored in memory 82. In some examples, the device test sequence(s) are triggered automatically (without further input from the programmer 130 or other device external to the IMD 126) after the one or more leads are connected to the IMD. In the device test sequence according to some examples, processor 80 causes IMD 126 to conduct, in series or in parallel, the following qualification tests:
(1) detecting an impedance for at least one electrical path that includes the at least one electrode to determine a connection status of the IMD to the at least one electrode and, in some examples, the connection of the electrode to the patient; and
(2) comparing EGM amplitudes of, for example, R-waves, over an EGM test period against a predetermined threshold.

In some examples, IMD 126 further performs a qualification test (3), which includes determining a pacing capture threshold (PCT) for the IMD. Processor 80 may determine whether a PCT threshold is adequate, e.g., below a threshold. In some examples, the IMD 126 detects a signal indicative of capture during the PCT test and/or a clinician monitors the surface ECG of the patient to determine electrical activity of heart associated with pacing capture.

In some examples, the signal generator 84 paces the heart of the patient prior to, during, and/or after performing any of the qualification tests (1)-(3). In some examples, the pacing is in VOO mode (asynchronous ventricular pacing), and in some cases the pacing is in a VVI mode (ventricular demand pacing), but any form of ventricular or atrial pacing may be used. In some examples, IMD 126 operates in OVO mode (no chambers of the heart paced, ventricular sensing only).

Once one or more of the qualification tests (1)-(3) are completed in a passing range, in some examples IMD 126 generates an output signal indicating that the qualification tests are complete. In some examples, IMD 126 generates no output signal, but a clinician may observe on a surface ECG that pacing capture threshold (PCT) in qualification test (3) has been successfully performed by the IMD, e.g., based on observing an ECG signal reflecting successful capture of heart by the pacing delivered by IMD 126. Based on knowledge that successful capture completes the test sequence, the clinician may determine qualification tests have been successfully completed by IMD 126.

In qualification test (1), sensing module 86 and/or processor 80 are capable of collecting, measuring, and/or calculating impedance data according to impedance parameters 81 stored in memory 82 for any of a variety of electrical paths that include two or more of the electrodes 124A-C. Sensing module 86 may include an impedance measurement module 92 with circuitry configured to measure electrical parameter values during delivery of an electrical signal between at least two of the electrodes. Processor 80 may determine impedance values based on parameter values measured by the impedance measurement module 92, and store the measured impedance values in the memory 82.

In some examples, processor 80 may perform an impedance measurement by controlling delivery, from the signal generator 84, of a voltage pulse or other voltage-controlled waveform between selected first and second electrodes 124. The impedance measurement module 92 may measure a resulting current, and the processor 80 may calculate a resistance based upon the voltage amplitude of the pulse and the measured amplitude of the resulting current. In other examples, the processor 80 may perform an impedance measurement by controlling delivery, from the signal generator 84, of a current pulse or other current-controlled waveform between first and second electrodes, the measurement module 92 may measure a resulting voltage, and the processor 80 may calculate a resistance based upon the current amplitude of the pulse and the measured amplitude of the resulting voltage. The measurement module 92 may include circuitry for measuring amplitudes of resulting currents or voltages, such as sample and hold circuitry.

In these examples, signal generator 84 delivers signals that do not necessarily deliver stimulation therapy to the heart 122, due to, for example, the amplitudes of such signals and/or the timing of delivery of such signals. For example, these signals may include sub-threshold amplitude signals that may not stimulate the heart 122. In some cases, these signals may be delivered during a refractory period, in which case they also may not stimulate the heart 122. IMD 126 may use defined or predetermined pulse amplitudes, widths, frequencies, or electrode polarities for the pulses delivered for these various impedance measurements.

In certain cases, IMD 126 may measure impedance values that include both a resistive and a reactive (i.e., phase) component. In such cases, IMD 126 may measure impedance during delivery of a sinusoidal or other time varying signal by signal generator 84, for example. Thus, as used herein, the term "impedance" is used in a broad sense to indicate any collected, measured, and/or calculated value that may include one or both of resistive and reactive components.

Processor 80 may control a plurality of measurements of the impedance of any one or more electrical paths including combinations of electrodes 124A-C according to the impedance parameters 81 stored therein. In some examples, the processor 80 determines that the qualification test (1) is a pass when processor 80 determines that the impedance measurement module 92 detects an impedance threshold stored in the memory 82. In some examples, an impedance measurement of about 300 Ω to about 2000 Ω, or about 300 Ω to about 1000 Ω, is sufficient to provide a pass for qualification test (1).

In some examples, before and during IMD 126 performing the qualification test (1), signal generator 84 paces the heart in VOO mode (ventricular pacing, no sensing). For example, signal generator 84 paces the heart at about 80 beats per minute (bpm). In some examples, signal generator 84 and sensing module 86 may be used to pace the heart in VVI mode (sensed ventricular pacing) prior to the IMD performing the qualification test (1). For example, signal generator 84 may pace the heart at about 60 bpm in response to absence of detection of signals (e.g., detection of the absence of intrinsic R-waves) by sensing module 86. In some examples, the heart may be paced in VOO or VVI mode in parallel with the IMD performing the impedance measurements in qualification test (1).

Processor 80 may control signal generator 84 to deliver the pacing pulses during or after impedance measurement according to the qualification test parameters 83 stored in the memory 82. For example, processor 80 may control the timing or amplitude of test pulses based on the qualification test parameters 83 which, in some examples, can specify a period of time, e.g., a window, subsequent a pacing pulse or detected cardiac event, which may be an R-wave or a P-wave, or other EGM measurement, noise, an asystolic EGM signal, or the like, in which one or more impedance measurement pulses may be delivered. In some examples, the duration of the period may be selected as appropriate to determine the most accurate impedance values. Furthermore, by controlling the timing of impedance measurement pulses in this manner, IMD 126 may avoid interference with the accuracy of impedance measurements by intrinsic cardiac signals. Processor 80 may compare the impedances measured from each of the test pulses to an impedance threshold, and evaluate the connection and integrity of lead 112 based on the comparison. In some examples, processor 80 may also switch from a current sensing configuration to an alternative sensing or therapy configuration in response to determining a lead related condition or other integrity issue with a configuration.

In some examples, IMD 126 repeatedly performs the qualification test (1) until the processor 80 determines a passing impedance measurement over a predetermined time period such as, for example, 2 minutes, 5 minutes, 15 minutes, 30 minutes, 45 minutes or 1 hour. If the measured impedance during the predetermined time period is not within a predetermined passing range, qualification test (1) is determined by the processor 80 to be a failure, and the result is optionally stored in the memory 82. In some examples, the predetermined passing range is about 300 Ω to about 2000 Ω, or about 300 Ω to about 1000 Ω, but the passing range may be set at any appropriate level. If the measured impedance in qualification test (1) is within the passing range, the qualification test (1) is determined by the processor 80 to be a pass, and the result may optionally be stored in the memory 82. If the qualification test (1) is determined to be a pass, in some examples the processor 80 may cause the signal generator 84 and the sensing module 86 to initiate pacing of the heart in, for example, VVI mode, prior to initiation of qualification tests (2) or (2)-(3). For example, in some cases, the pacing is applied by the IMD to the heart at 60 bpm at a pacing output of about 5 V.

In some examples, the following completion of qualification test (1), processor 80 controls IMD 126 to perform qualification test (2). In qualification test (2), processor 80 may detect aspects of EGM signals sensed by sensing module 86 such as, for example, R-waves, P-waves, and COI parameters, according to test criteria 85 stored within the memory 82. In some examples, the EGM test criteria 85 may include one or more R-wave or P-wave amplitude thresholds, to which the processor 80 may compare amplitudes of sensed R-waves and P-waves. As an example, the suitable EGM threshold may be satisfied by measurable R-waves having an amplitude of at least about 5 mV. If the EGM threshold(s) are satisfied, the qualification test (2) is completed and the processor 80 initiates the optional qualification test (3). If the heart rate is too fast, e.g., whether or not the EGM threshold is determined to be a pass, the processor may repeat one or both of qualification tests (1) and (2), in some examples.

In some examples, if processor 80 and sensing module 84 are unable to detect a threshold number of R-waves or other EGM components satisfying the threshold amplitude, processor 80 may lower the pacing rate, e.g., lengthen the escape interval, to allow more intrinsic conduction. For example, processor 80 may decrement the pacing rate, e.g., by steps or otherwise, to minimum rate, e.g., 40 beats per minute (bpm). If the EGM sensing still does not pass the test, processor may change the EGM sensing parameters, e.g., amplitude threshold or electrodes 124 used for sensing.

In some examples, processor 80 may perform COI tests according to COI test parameters 85 in the memory 82, following the completion of the EGM qualification test (2), or in parallel with the EGM qualification test (2). In some examples, sensing module 86 may sense the heart in, for example, OVO mode, prior to initiation of the COI test parameters stored in the memory 82. Suitable COI parameters 85 in the memory 82 include, but are not limited to, determination of: the maximum amplitude of the ST segment in the EGM of the patient, amplitude of the ST segment 80 milliseconds (ms) from the segment's beginning, the area under the wave curve (from R-wave start to the end of the ST segment), the area under the ST segment, amplitude at a start of ST segment, median and quartile amplitudes of the first 200 ms following ST segment, amplitude of the R wave, duration of the ST segment, duration of the signal (QT), ratio of R-wave amplitude to maximum amplitude of ST segment, ratio of R-wave amplitude to amplitude of ST segment 80 ms from start, and combinations thereof.

In some examples, processor 80 may determine the values of COI parameters based on an analysis of a digitized version of the EGM signal. For example, processor 80 may perform a deconvolution operation of the time-series of EGM samples to recover lower frequency COI content, e.g., emphasize features in the EGM indicative of COI. In some examples, the other EGM signals such as R-wave or P-wave amplitude may be evaluated over the same time period that the COI parameters are determined. If insufficient COI is detected, or if insufficient EGM amplitudes are detected, the qualification test (2) may be terminated by the processor 80.

If processor 80 determines that IMD 126 passes qualification test (2), processor 80 optionally initiates qualification test (3) to evaluate the PCT of IMD 126 according to the PCT/VCM (ventricular capture management) parameters 87 in the memory 82. In the PCT qualification test, VCM module 89, sensing module 86, and signal generator 84 operate according to PCT parameters stored in the memory 82 to evaluate the energy required to cause depolarization and contraction of the heart tissue of the patient, and compares the required energy to a PCT threshold. For example, in some embodiments, the PCT threshold may be evaluated by VCM module 89 to automatically monitor pacing thresholds at periodic intervals. Once the pacing threshold is determined, in some examples VCM module 89 determines a target pacing output based on a predetermined safety margin and a predetermined minimum EGM amplitude.

In some examples, processor 80 may run abbreviated VCM tests in which the PCT capture threshold is required to be less than about 2.5 V. In some examples, the processor 80 initiates the signal generator 84 to pace the heart in VVI mode for a predetermined time period (for example, 30 seconds to 60 seconds) to evaluate PCT capture threshold. In some examples, the heart is paced in VVI mode at about 90 bpm to determine the PCT capture threshold. Such a pacing rate is likely faster than the intrinsic heart rate of the patient so that intrinsic heart beats will not interfere with pacing capture threshold testing.

If the PCT capture threshold is not achieved, the processor 80 indicates that qualification test (3) was not successful, and the result may optionally be stored in the memory 82. If the PCT capture threshold is achieved and qualification test (3) is determined to be a pass, in some examples processor 80 may generate a confirmation signal, and a result is optionally stored in the memory 82. Suitable indications include, for example, energizing a LED, an audible alert, or sending a confirmation signal to an optional programmer 130. In some examples, processor 80 provides no indication of the status of the qualification test (3), and pacing capture may be evaluated by a clinician as pacing at a fixed rate visible to the clinician on a surface ECG monitor.

In some examples, if qualification test (3) is determined to be a pass, IMD 126 switches back to pacing in, for example, OVO mode, for a predetermined period of time (for example, about 6 seconds to about 10 seconds) to allow intrinsic conduction so that sensing module 86 may measure COI parameters. The COI parameters may then be compared to previously measured COI values stored in memory 82, and if a COI percent change threshold is met, the processor 80 provides an indication that the qualification test (3) and the COI parameters were successful. If the COI percent change threshold fails, the processor 80 instructs the signal generator 84 and the sensing module 86 to switch to pacing in, for example, VVI mode, for a predetermined period of time such as, for example, about 60 seconds.

After a selected pacing time of, for example, about 60 seconds, processor 80 may instruct the signal generator 84 and the sensing module 86 to return to OVO mode and the COI parameters are again evaluated and compared the COI values previously stored in memory 82. For example, the COI values may be compared to stored COI values previously stored in memory 82 from COI measurements completed by IMD 126 minutes to 5 minutes earlier. If the COI percent change threshold is met, processor 80 generates an output indicating that the qualification test (3) and the COI determination were a pass. Otherwise, the processor 80 may output an indication that the COI parameters were not satisfied.

The various components of the IMD 126 are coupled to a power source 90, which may include a rechargeable or non-rechargeable battery. A non-rechargeable battery may be capable of holding a charge for several years, while a rechargeable battery may be inductively charged from an external device, e.g., on a daily or weekly basis.

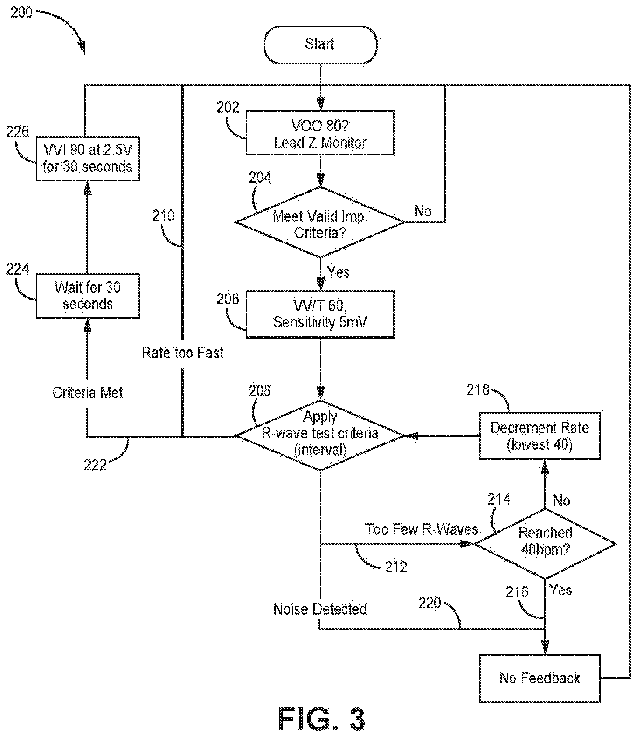

FIG. 3 is a flow diagram illustrating an example of a device test sequence 200 performed by an IMD 126 (for example, FIG. 1) according to the present disclosure. As shown in FIG. 3, after IMD 126 detects attachment of lead 112, processor 80 triggers a device test sequence without input from an external device such as a programmer. IMD 126 may detect attachment of lead 112 based on measuring an impedance consistent with connection to lead 112.

Prior to or at the same time the device test sequence initiates, the signal generator 84 (FIG. 2) paces the ventricle of the heart in VOO mode at a rate of about 80 pulses per minute, and the impedance measurement module 92 performs one or more impedance measurements of the qualification test (1) after each paced event to determine if the measured lead impedance is within a passing range (202). The one or more impedances within the passing range may indicate one or more of attachment of IMD 126 to lead 112, integrity of lead 112, and attachment of lead 112 to the heart. If the qualification test (1) is determined to be a pass (YES of 204), processor 80 controls IMD 126 to proceed to qualification test (2). If the measured impedance is outside the passing range (NO of 204), the qualification test (1) is deemed to fail, and IMD 126 returns to step 202 to re-initiate the qualification test (1) and search for a passing lead impedance value. As noted above, IMD 126 may continue the qualification test (1) for a predetermined period of time such as, for example, about 30 minutes, or indefinitely until qualification test (1) is determined to be passed (e.g., indicating lead connection and that other qualification tests may proceed).

If qualification test (1) is a pass (YES of 204), processor 80 triggers the signal generator 84 and the sensing module 86 to perform pacing of the ventricle of the heart of the patient in VVI mode at a rate of 60 bpm and with an R-wave sensitivity of 5 mV (206). Processor 80 then initiates the qualification test (2) by applying R-wave test criteria 85 stored in the memory 82 (208). If the measured intrinsic heart rate exceeds a threshold (210), processor 80 may return to the VOO pacing (202) and re-runs qualification test (1) (204). If the R-wave criteria are not met due to too few measured R-waves (212), processor 80 determines whether the pacing rate has already been decremented to a minimum value, e.g., 40 bpm (214). If so (YES of 214), processor 80 may return IMD 126 to qualification test (1) (202,204) without providing feedback of a successful test. If the pacing rate has not yet been decremented to the minimum value (NO of 214), processor 80 decrements the pacing rate (218) and again attempts to detect and measure R-waves until the R-wave test criteria 85 are satisfied (208). If excessive processor 80 determines that an amount of noise detected in the EGM signal detected by sensing module 86 during qualification test (2) (220), the processor may return to the qualification test (1) (202, 204).

If the measured EGM amplitudes satisfy the EGM test criteria 85 (222), the qualification test (2) is deemed a pass. Processor 80 may delay for 30 seconds (224), and then configured the sensing module 86 and the signal generator 84 to apply pacing to the heart in VVI mode at 90 bpm and 2.5 V for 30 seconds (226). A clinician may then observe a surface ECG monitor to confirm proper pacing and device capture. The operation of FIG. 3 thus provides a rapid and simple check on the success of the IMD implantation procedure.

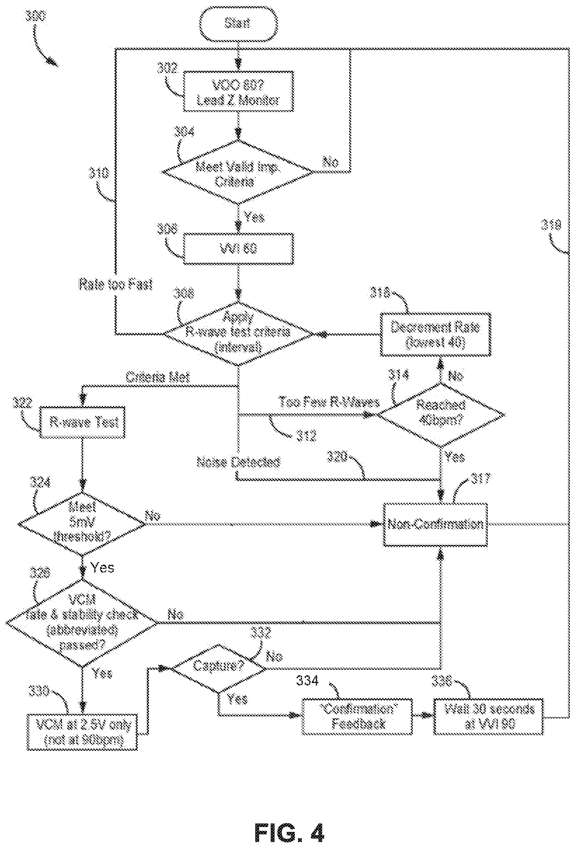

FIG. 4 is a flow diagram illustrating another example of a device test sequence 300 performed by an IMD 126 (for example, FIG. 1) according to the present disclosure. As shown in FIG. 4, after attachment of at least one lead 112 to IMD 126, processor 80 triggers a device test sequence without input from an external device such as a programmer. IMD 126 may detect attachment of lead 112 based on measuring an impedance consistent with connection to lead 112.

According to the device test sequence, processor 80 controls signal generator 84 (FIG. 2) to pace the heart in VOO mode at a rate of about 80 pulses per minute (302) and, e.g., on every paced event, impedance measurement module 92 to measure an impedance to perform the qualification test (1) to determine if the measured lead impedance is within a passing range (304). The one or more impedances within the passing range may indicate one or more of attachment of IMD 126 to lead 112, integrity of lead 112, and attachment of lead 112 to the heart. If the qualification test (1) is determined to be a pass (YES of 304), processor 80 controls IMD 126 to proceed to qualification test (2). If the measured impedance is outside the passing range (NO of 304), the qualification test (1) is deemed a failure and processor 80 re-initiates the qualification test (1) and searches for a passing lead impedance value (302,304). IMD 126 may continue the qualification test (1) for a predetermined period of time such as, for example, about 30 minutes, or indefinitely.

If qualification test (1) is a pass (YES of 304), processor 80 triggers signal generator 84 and the sensing module 86 to pace the heart of the patient in VVI mode at a rate of 60 bpm (306). Processor 80 may then initiate the qualification test (2) by applying R-wave test interval criteria 85 stored in the memory 82 to the EGM sensing by sensing module 86 (308). If the measured intrinsic heart rate is determined to be too high, e.g., by exceeding a heart rate threshold (310), processor 80 returns to the VOO pacing (302), re-runs qualification test (1) (304).

If the R-wave criteria are not met due to too few measured R-waves (312), processor 80 determines whether the pacing rate has already been decremented to a minimum value, e.g., 40 bpm (314). If so (YES of 314), processor 80 may return IMD 126 to qualification test (1) (302, 304) without confirmation to the clinician or with a signal of non-confirmation (317). If the pacing rate has not yet been decremented to the minimum value (NO of 314), processor 80 may decrement the pacing rate (318) and sensing module 86 may again attempt to detect and measure R-waves until the EGM test criteria 85 are satisfied (308). If sensing module 86 detects an amount of noise in the sensed EGM greater than a threshold (320), processor 80 may return IMD 126 to the qualification test (1) (302, 304) without confirmation to the clinician or with a non-confirmation signal to the clinician (317).

If the R-wave interval criteria 85 are satisfied (YES of 308), processor 80 applies an R-wave amplitude test from the EGM test criteria 85 (322), and determines whether the R-waves meet the criteria, e.g., whether a sufficient number of R-waves were sensed when sensing module 86 applies a threshold of 5mV (324). If the sensed R-waves meet the criteria (YES of 324), the qualification test (2) is deemed a pass and processor 80 may proceed qualification test (3) (326). If the sensed R-waves do not meet the 5 mV criteria (NO of 324), processor 80 may return IMD 126 to the qualification test (1) (302, 304) without confirmation to the clinician (317).

In qualification test (3), processor 80 may initially perform a rate and stability check (326). If the check is passed (YES of 326), processor 80 may control VCM module 89 to perform an abbreviated VCM test to determine whether capture occurs at or below 2.5V (330). In capture is confirmed (YES of 332), processor 80 may control IMD 126 to provide a confirmation signal (334), which may include pacing at VVI 90 for 30 seconds (336). If the VVI pacing protocol is successful as observed on a surface ECG, the qualification test (3) is deemed a pass, and the implant of the IMD is deemed to be successful. If the PCT is outside the target range at 2.5 V (NO of 332) or the rate and stability check is not passed (NO of 326), the VCM module re-initiates the VCM test, or after a predetermined number of attempts processor 30 controls IMD to provide a non-confirmation signal or returns IMD 126 to the qualification test (1) (302, 304) without confirmation to the clinician (317).

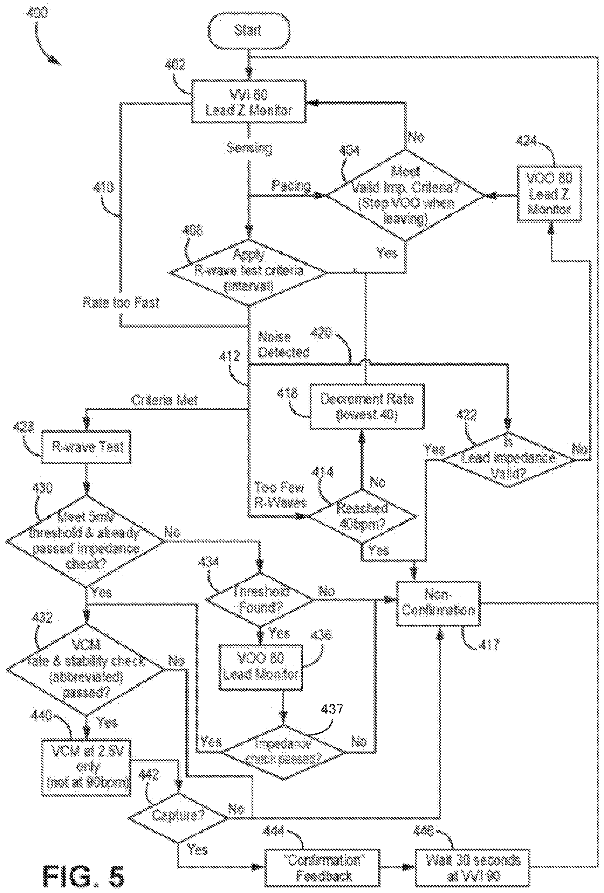

FIG. 5 is a flow diagram illustrating another example of a device test sequence 400 performed by an IMD 126 (for example, FIG. 1) according to the present disclosure. As shown in FIG. 5, after attachment of at least one lead to the IMD, the processor 80 triggers a device test sequence without input from an external device such as a programmer. IMD 126 may detect attachment of lead 112 based on measuring an impedance consistent with connection to lead 112.

According to the device test sequence, processor 80 controls signal generator 84 and sensing module 86 (FIG. 2) pace the heart in VVI mode at a rate of about 60 bpm (402). Based on beats being paced according to the VVI mode, processor 80 transitions IMD 126 to VOO pacing, and controls impedance measurement module 92 measure impedance to determine if the measured lead impedance is within a passing range (404). The one or more impedances within the passing range may indicate one or more of attachment of IMD 126 to lead 112, integrity of lead 112, and attachment of lead 112 to the heart. If the measured impedance does not satisfy the impedance criteria (N of 404), processor 80 may control IMD 126 to resume pacing according to the VVI mode.

If qualification test (1) is a pass (YES of 404), processor controls IMD 126 to return to the VVI mode. For intrinsic beats that are sensed during pacing in the VVI mode, processor 80 may control sensing module 86 to perform qualification test (2) by applying an interval measurement in the EGM test criteria 85 stored in the memory 82 for sensing intrinsic beats (408). If the measured heart rate is too high, e.g., above a threshold rate, processor 80 returns continues pacing in the VVI mode (410). If the R-wave criteria are not met due to too few intrinsic R-waves (412), processor 80 determines whether the pacing rate is at a minimum value, e.g., 40 bpm (414). If the pacing rate is not at the minimum (NO of 414), processor 80 may decrement the pacing rate (418) and continue VVI pacing and monitoring for intrinsic R-waves. If the pacing rate has already been decremented to 40 bpm (YES 414), processor 80 may provide a non-confirmation signal in step 417 that qualification test (2) is not a pass, and re-initiate qualification tests (1 and 2) (402, 404, and 408).

If processor 80 determines that an amount of noise in the EGM exceeds a threshold (420), the processor may control impedance measurement module 92 to measure impedance and determine whether the measurement is valid (422). If the measured lead impedance is valid (YES of 422), processor 80 may issue a non-confirmation signal 417 and re-initiate qualification tests (1 and 2) (402, 404, and 408). If the measured impedance is not valid (NO of 422), processor 80 may control IMD 126 to switch to VOO pacing, e.g., at a rate of 80 bpm (424), and determine whether the measured impedances meet impedance criteria 81 (404).

If the sensed R-waves satisfy interval criteria of test criteria 85, processor 80 applies an R-wave amplitude test from the EGM test criteria 85 (428). Processor 80 determines whether one or more R-wave amplitudes are greater than a threshold, e.g., 5 mV, and the impedance check from qualification test (1) is a pass (430). If criteria of qualification tests (1) and (2) are met (YES of 430), processor 80 proceeds to perform a capture management rate and stability check (432). If the measured R-wave amplitude(s) do not meet the 5 mV threshold (NO of 430 and 434), processor 80 causes IMD 126 to return the non-qualification signal (417), and may return to VVI pacing at 60 bpm (402). If the measured R-wave amplitude(s) do meet the 5 mV threshold, but the impedance check was not a pass (NO of 430 and YES 434), processor 80 may control IMD 126 to transition to VOO pacing at 80 bpm and control impedance measurement module 92 to measure impedances (436). If the impedance check is not a pass (NO of 437), processor 80 returns the non-confirmation signal (417), and may return to VVI pacing at 60 bpm (402). If the impedance check is a pass (YES of 437), processor 80 proceeds to perform a capture management rate and stability check (432).

If the rate and stability check is passed (YES of 432), processor 80 may control VCM module 89 to perform an abbreviated VCM test to determine whether capture occurs at or below 2.5V (440). Processor 80 sets ventricular pacing output to 2.5V and the PCT is evaluated by the VCM module 89. In capture is confirmed (YES of 442), processor 80 may control IMD 126 to provide a confirmation signal (444), which may include pacing at VVI 90 for 30 seconds (446). If the VVI pacing protocol is successful as observed on a surface ECG, the qualification test (3) is deemed a pass, and the implant of the IMD is deemed to be successful. If capture is not observed/detected at 2.5 V (NO of 442) or the rate and stability check is not passed (NO of 432), the VCM module re-initiates the VCM test, or after a predetermined number of attempts processor 80 controls IMD to provide a non-confirmation signal (417) or returns IMD 126 to the qualification tests (1 and 2) and VVI pacing (402) with a signal of confirmation to the clinician (417).

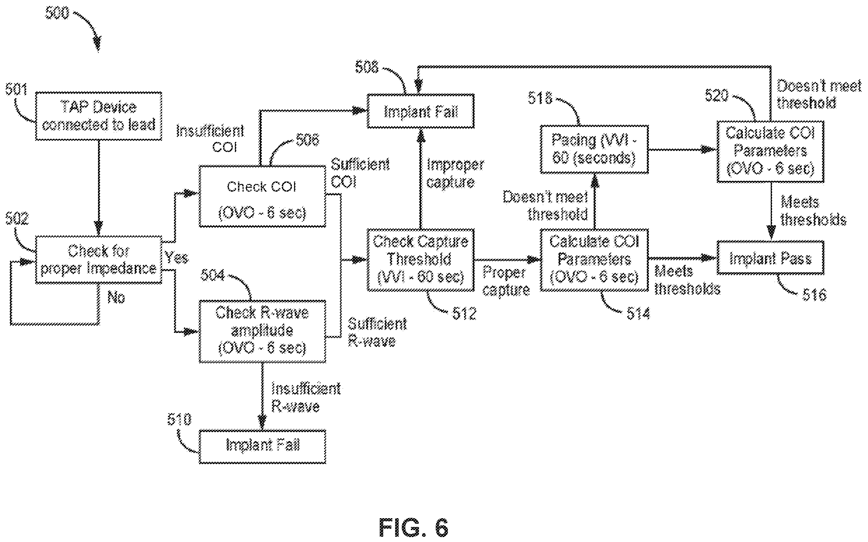

FIG. 6 is a flow diagram illustrating another embodiment of a device test sequence 500 performed by an IMD 126 (for example, FIG. 1) according to the present disclosure. IMD 126 is attached to lead 112 (501), and processor 80 triggers a device test sequence without input from an external device such as a programmer. According to the sequence 500, processor 80 controls impedance measurement module 92 to measure impedances according to qualification test (1) to determine if the measured lead impedance is within a passing range (502). The one or more impedances within the passing range may indicate one or more of attachment of IMD 126 to lead 112, integrity of lead 112, and attachment of lead 112 to the heart. If the qualification test (1) is determined to be a pass (YES of 502), processor 80 proceeds to qualification test (2) (504). If the measured impedance is outside the passing range (NO of 502), processor 80 may re-initiate the qualification test (1) and search for a passing lead impedance value (502). Processor 80 may continue the qualification test (1) for a predetermined period of time such as, for example, about 30 minutes, or indefinitely.

According to qualification test (2), processor 80 may control IMD 126 to enter an OVO mode and sensing module 86 to sense intrinsic R-wave amplitudes and COI parameters (504,506). As noted above, COI parameters 85 can include the maximum amplitude of the ST segment, the amplitude of the ST segment 80 ms from the segment's beginning, the area under the wave curve (from R-wave start to the end of the ST segment), the area under the ST segment, amplitude at a start of ST segment, median and quartile amplitudes of the first 200 ms following ST segment, amplitude of the R wave, duration of the ST segment, duration of the signal (QT), ratio of R-wave amplitude to maximum amplitude of ST segment, ratio of R-wave amplitude to amplitude of ST segment 80 ms from start, and combinations thereof. If insufficient COI is detected after an evaluation period of about 6 seconds, processor 80 outputs that the implant failed (508). If an insufficient R-wave amplitude is detected, e.g., after an evaluation period of 6 seconds, processor 80 outputs that the implant failed (510).

Otherwise, processor 80 controls IMD 126 to switch to pacing in VVI mode for 60 seconds to evaluate pacing capture threshold (PCT) in qualification test (3) (512). If there is not proper capture as measured by the VCM module 89, processor outputs that implant has failed (508). If there is proper capture, processor 80 controls IMD 126 to switch back to OVO mode for a period of 6 seconds to calculate COI parameters (514). The COI parameter values are compared to the values from a time period before current values, such as 1 minute, 5 minutes, or 10 minutes. If the COI percent change threshold is met, processor 80 outputs that the implant passed (516). If the COI percent change threshold fails, processor 80 controls IMD 126 to pace in VVI mode for 60 seconds (518).

After 60 seconds of pacing, processor 80 returns IMD 126 to OVO mode to calculate COI parameters again (520). The parameters are compared to the initial values from 2 minutes before. If the COI percent change threshold is met, processor 80 outputs that the implant passed (516). Otherwise, processor 80 outputs that the implant failed (508).

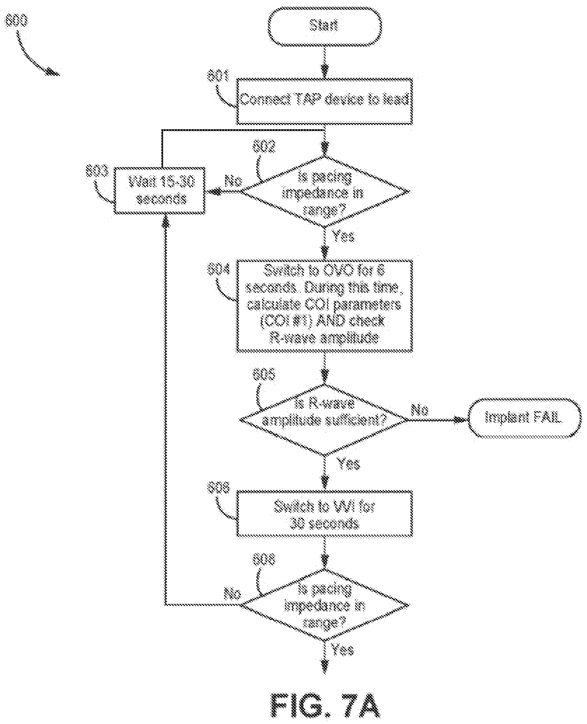

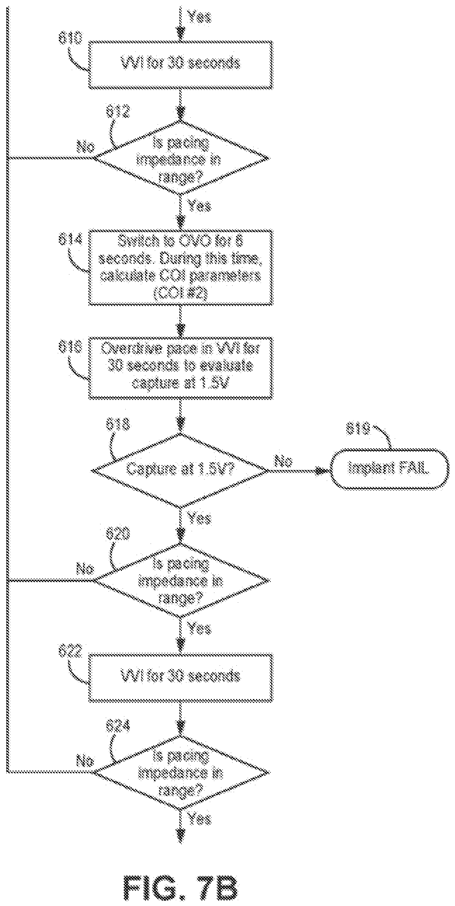

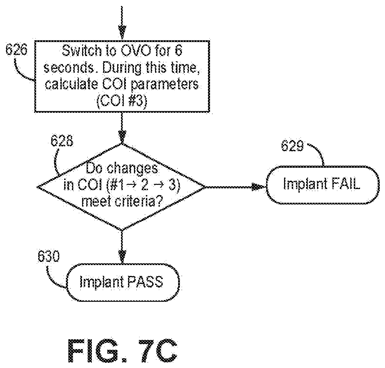

FIGS. 7A-7C are a flow diagrams illustrating another example of a device test sequence 600 performed by an IMD 126 (for example, FIG. 1) according to the present disclosure. After attachment of at least one lead 112 to IMD 126 (601), the processor 80 triggers a device test sequence without input from an external device such as a programmer. While in a single chamber asynchronous mode such as VOO or a single chamber demand pacing mode such as VVI, processor 80 controls impedance measurement module 92 to measure impedances to perform the qualification test (1) to determine if the measured lead impedance is within a passing range (602). The one or more impedances within the passing range may indicate one or more of attachment of IMD 126 to lead 112, integrity of lead 112, and attachment of lead 112 to the heart. If the measured impedance is outside the passing range (NO of 602), the qualification test (1) is deemed to be a failure, and processor 80 may, after a delay (603) re-initiate the qualification test (1) and search for a passing lead impedance value (602). Processor 80 may repeat performance of qualification test (1) after an impedance test interval of about 15 seconds to about 30 seconds. Processor 80 may continue the qualification test (1) for a predetermined period of time such as, for example, about 30 minutes, or indefinitely.

If the qualification test (1) is determined to be a pass (YES of 602), processor 80 may switch IMD 126 to operation in the OVO mode, and the sensing module 86 may sense intrinsic R-wave amplitudes and COI parameters for qualification test (2) (604). As noted above, these COI parameters 85 can include determining any or all of the following: the maximum amplitude of the ST segment, the amplitude of the ST segment 80 ms from the segment's beginning, the area under the wave curve (from R-wave start to the end of the ST segment), the area under the ST segment, amplitude at a start of ST segment, median and quartile amplitudes of the first 200 ms following ST segment, amplitude of the R wave, duration of the ST segment, duration of the signal (QT), ratio of R-wave amplitude to maximum amplitude of ST segment, ratio of R-wave amplitude to amplitude of ST segment 80 ms from start, and combinations thereof.

If R-wave amplitude sensing is determined to be insufficient (NO of 605), qualification test (2) is determined to be a failure. If R-wave amplitude sensing is determined to be sufficient (YES of 605), processor 80 switches IMD 126 to pacing in VVI mode for a period of time such as 30 or 60 seconds (606), and proceeds to perform the impedance check of qualification test (1) (608). To avoid intrinsic cardiac interference, in some examples VOO pacing may performed instead of VVI pacing for this impedance measurement. If the impedance check of qualification test (1) is determined to be out of a passing range (NO of 608), processor 80 assumes that a lead is being repositioned or reconnected to the IMD and waits (603) prior to performing another impedance check (602).

If the impedance is within a passing range (YES of 608), processor 80 again implements VVI pacing for 30 seconds (610) and re-checks impedance (612). If the impedance is in a passing range (YES of 612), processor 80 switches to OVO mode for a period of about 6 seconds, and calculates any or all of the COI parameters discussed above with respect to step 604 (614).

Processor 80 controls IMD 126 to initiate overdrive pacing in VVI mode for 30 seconds to evaluate device capture at 1.5 V for PCT qualification test (3) (616). If there is not proper capture at 1.5V as measured by the VCM module 89 (NO of 618), processor 80 outputs that the implant failed (619). If there is proper capture (YES of 618), processor 80 again controls performance of impedance measurements for qualification test (1) (620). If the impedance is within a predetermined passing range (YES of 620), processor 80 controls IMD 126 to pace the heart in VVI mode for 30 seconds (622), and then again checks impedance (624).

If the impedance remains in the passing range (YES of 624), processor 80 switches IMD 126 back to OVO mode for a period of 6 seconds to calculate COI parameters (626). Processor 80 compares the COI parameters to the previously measured COI values from steps 604 and 614 (628). If the COI percent change threshold is met (YES of 628), processor outputs an indication that the implant passed (630). If the COI percent change threshold is not met (NO of 628), processor 80 may indicate that the IMD implantation failed (629).

It should be noted that the therapy system 100 may not be limited to treatment of a human patient. In alternative examples, the therapy system 100 may be implemented in non-human patients, e.g., primates, canines, equines, pigs, and felines. These other animals may undergo clinical or research therapies that my benefit from the subject matter of this disclosure.

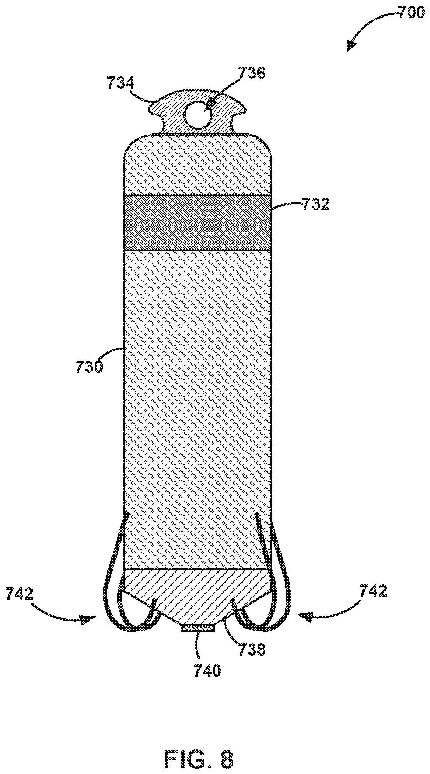

FIG. 8 is a conceptual drawing illustrating an example configuration of another IMD 700, which may be configured to automatically trigger performance of diagnostic self-tests and provide rapid feedback to the practitioner to support a procedure to implant the IMD, e.g., in the manner described herein with respect to IMD 126 and FIGS. 4-7B.

As shown in FIG. 8, IPD 700 includes case 730, cap 738, electrode 740, electrode 732, fixation mechanisms 742, flange 734, and opening 736. Together, case 730 and cap 738 may be considered the housing of IMD 700. In this manner, case 730 and cap 738 may enclose and protect the various electrical components, e.g., a processor, signal generator, sensing module, impedance measurement circuitry, VCM module, memory, telemetry module, and other circuitry as described with respect to FIG. 2, within IMD 700. IMD 700 may a plurality of electrodes (e.g., electrodes 732 and 740) for delivery and sensing of electrical signals.

Electrodes 732 and 740 are carried on the housing created by case 730 and cap 738. In this manner, electrodes 732 and 740 may be considered leadless electrodes, and IMD 700 may be considered a leadless IMD, e.g., a leadless pacemaker or leadless pacing device. In the example of FIG. 8, electrode 740 is disposed on the exterior surface of cap 738. Electrode 740 may be positioned to contact cardiac tissue upon implantation. Electrode 732 may be a ring or cylindrical electrode disposed on the exterior surface of case 730. Both case 730 and cap 738 may be electrically insulating.

Electrode 740 may be used as a cathode and electrode 732 may be used as an anode, or vice versa, for delivering cardiac pacing such as bradycardia pacing, CRT, ATP, or post-shock pacing. However, electrodes 732 and 740 may be used in any stimulation configuration. In addition, electrodes 732 and 740 may be used to detect intrinsic electrical signals, e.g., from cardiac muscle.

Fixation mechanisms 742 may attach IMD 700 to tissue, e.g., cardiac tissue. Fixation mechanisms 742 may be active fixation tines, screws, clamps, adhesive members, or any other mechanisms for attaching a device to tissue. As shown in the example of FIG. 8, fixation mechanisms 742 may be constructed of a memory material, such as a shape memory alloy (e.g., nickel titanium), that retains a preformed shape. During implantation, fixation mechanisms 742 may be flexed forward to pierce tissue and allowed to flex back towards case 730. In this manner, fixation mechanisms 742 may be embedded within the target tissue. Flange 734 may be provided on one end of case 730 to enable tethering or extraction of IMD 700.

IMD 700, e.g., processor 80 of IMD 700, may be configured to detect placement of the IMD into a patient and, in response to placement of the IMD into the patient, to initiate a device test sequence, comprising any of the a plurality of qualification tests disclosed herein, over an evaluation period. Processor 80 may be configured to detect positioning of IMD 700 within patient based on a change in impedance measured by impedance measurement circuitry 92 via electrodes 732 and 740 when the electrodes are exposed to blood and/or come into contact with tissue. For the test sequence, processor 80 may control IMD 700 to perform qualification tests including measurement of impedance, comparison of EGM amplitudes to a threshold, evaluation of pacing capture, determining COI parameters, or any one or more of the tests described herein.

The techniques described in this disclosure, including those attributed to the IMDs 126 and 700, the programmer 130, or various constituent components, may be implemented, at least in part, in hardware, software, firmware or any combination thereof. For example, various aspects of the techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components, embodied in programmers, such as physician or patient programmers, stimulators, image processing devices or other devices. The term "processor" or "processing circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

When implemented in software, the functionality ascribed to the systems, devices and techniques described in this disclosure may be embodied as instructions on a computer-readable medium such as random access memory (RAM), read-only memory (ROM), non-volatile random access memory (NVRAM), electrically erasable programmable read-only memory (EEPROM), FLASH memory, magnetic data storage media, optical data storage media, or the like. The instructions may be executed to support one or more aspects of the functionality described in this disclosure.

Various examples have been described.

## Claims

1. An implantable medical device, IMD, configured to be coupled to at least one implantable medical lead (112), wherein the IMD (126) comprises:
sensing circuitry configured to sense an electrogram, EGM, signal of a patient via at least one electrode of the implantable medical lead;
impedance measurement circuitry (92) to measure impedance via the implantable medical lead; and
a processor (80) configured, in response to coupling of the IMD to the at least one implantable medical lead, to initiate a device test sequence comprising a plurality of qualification tests over an evaluation period in which the processor:
(1) controls the impedance measurement circuitry to measure an impedance for at least one electrical path that includes the at least one electrode to determine a connection status of the IMD to the at least one electrode; and
(2) compares EGM amplitudes of the patient over an EGM test period against a predetermined threshold.

2. The IMD of claim 1, wherein the processor (80) controls the sensing circuitry to measure current of injury, COI, parameters in the EGM following qualification test (1).

3. The IMD of claim 2, wherein the current of injury, COI, parameters include one or more of a maximum amplitude of an ST segment, an amplitude of the ST segment 80 milliseconds from a beginning of the segment, an area under a wave curve from an R-wave start to the end of the ST segment, an area under the ST segment, an amplitude at a start of the ST segment, median and quartile amplitudes of the EGM following the ST segment, a amplitude of an R-wave, a duration of the ST segment, a duration of the signal, QT, a ratio of the amplitude of the R-wave to maximum amplitude of ST segment, or a ratio of the amplitude of the R-wave to amplitude of ST segment 80 ms from start.

4. The IMD of claim 2 or 3, wherein the processor controls the sensing circuitry to measure the COI parameters in parallel with qualification test (2).

5. The **IMD** of any one or more of claims 1 to 4, wherein the processor (80) automatically initiates the device test sequence, without input from an external programmer, when the at least one implantable lead is connected to the IMD.

6. The IMD of any one or more of claims 1 to 5, wherein the evaluation period is about 2 minutes to about 1 hour following attachment of the at least one lead to the IMD.

7. The IMD of any one or more of claims 1 to 6, further comprising a signal generator (84) configured to deliver pacing pulses via the implantable medical lead, wherein the processor controls the signal generator to deliver pacing pulses in an asynchronous mode during qualification test (1) and a demand pacing mode during performance of the qualification test (2).

8. The IMD of any one or more of claims 1 to 7, wherein, for qualification test (2), the processor (80) is configured to:
determine that a threshold number of R-waves have not been sensed; and
reduce a rate of the pacing pulses based on the determination.

9. The IMD of any one or more of claims 1 to 6, further comprising a signal generator (84) configured to deliver pacing pulses via the implantable medical lead, wherein the device test sequence further comprises a qualification test (3) in which the processor controls the signal generator and the sensing circuitry to determine whether a pacing capture threshold, PCT, satisfies one or more criteria.

10. The IMD of claim 9, wherein the processor (80) controls performance of qualification tests (2)-(3) following qualification test (1).

11. The IMD of claim 9 or 10, wherein the processor (80) controls the sensing circuitry to measure current of injury, COI, parameters in the EGM after qualification test (3).

12. The IMD of claim 9 or 10, wherein the processor (80) controls performance of qualification test (3) at the end of the device test sequence, and pacing capture is observable by a clinician via an electrocardiogram monitor as indication of device test sequence success.

13. The IMD of any one or more of claims 1 to 12, wherein the processor (80) controls the IMD to generate a confirmation signal in response to success of the device test sequence.

14. The IMD of claim 13, further comprising a signal generator (84) configured to deliver pacing pulses via the implantable medical lead, wherein the confirmation signal comprises the delivery of pacing pulses.

15. A leadless implantable medical device, IMD, comprising:
a housing (730, 738) configured for implantation within a patient;
a plurality of electrodes (730, 740) on the housing;
sensing circuitry within the housing, the sensing circuitry configured to sense an electrogram, EGM, signal of the patient via the electrodes;
impedance measurement circuitry (92) within the housing, the impedance measurement circuitry configured to measure impedance via the electrodes; and
a processor (80) configured to detect placement of the IMD (700) into the patient and, in response to placement of the IMD into the patient, to initiate a device test sequence comprising a plurality of qualification tests over an evaluation period in which the processor:
(1) controls the impedance measurement circuitry to measure an impedance for at least one electrical path between the electrodes; and
(2) compares EGM amplitudes of the patient over an EGM test period against a predetermined threshold.

## Patentansprüche

1. Implantierbare medizinische Vorrichtung, IMD, die konfiguriert ist, um mit mindestens einem implantierbaren medizinischen Lead (112) gekoppelt zu werden, wobei die IMD (126) umfasst:
eine Erfassungsschaltung, die konfiguriert ist, um ein Elektrogrammsignal, EGM-Signal, eines Patienten über mindestens eine Elektrode des implantierbaren medizinischen Leads zu erfassen;
eine Impedanzmessschaltung (92), um eine Impedanz über das implantierbare medizinische Lead zu messen; und
einen Prozessor (80), der konfiguriert ist, um, als Reaktion auf das Koppeln der IMD mit dem mindestens einen implantierbaren medizinischen Lead, eine Vorrichtungstestsequenz zu initiieren, umfassend eine Vielzahl von Qualifizierungstests über einen Auswertungszeitraum, in dem der Prozessor:
(1) die Impedanzmessschaltung steuert, um eine Impedanz für mindestens einen elektrischen Pfad zu messen, der die mindestens eine Elektrode, um einen Verbindungsstatus der IMD mit der mindestens einen Elektrode zu bestimmen, einschließt; und
(2) EGM-Amplituden des Patienten über einen EGM-Testzeitraum mit einer zuvor bestimmten Schwelle vergleicht.

2. IMD nach Anspruch 1, wobei der Prozessor (80) die Erfassungsschaltung steuert, um Verletzungsstromparameter, COI-Parameter, in dem EGM nach dem Qualifizierungstest (1) zu messen.

3. IMD nach Anspruch 2, wobei die Verletzungsstromparameter, COI-Parameter, eines oder mehrere von einer maximalen Amplitude eines ST-Segments, einer Amplitude des ST-Segments 80 Millisekunden ab einem Beginn des Segments, einem Bereich unter einer Wellenkurve von einem R-Zacken-Start bis zu dem Ende des ST-Segments, einem Bereich unter dem ST-Segment, einer Amplitude an einem Start des ST-Segments, Median- und Quartil-Amplituden des EGM nach dem ST-Segment, einer Amplitude einer R-Zacke, einer Dauer des ST-Segments, einer Dauer des Signals, QT, einem Verhältnis der Amplitude der R-Zacke zu der maximalen Amplitude des ST-Segments oder einem Verhältnis der Amplitude der R-Zacke zu der Amplitude des ST-Segments 80 ms ab Start einschließen.

4. IMD nach Anspruch 2 oder 3, wobei der Prozessor die Erfassungsschaltung steuert, um die COI-Parameter parallel zu dem Qualifizierungstest (2) zu messen.

5. IMD nach einem oder mehreren der Ansprüche 1 bis 4, wobei der Prozessor (80) die Vorrichtungstestsequenz ohne Eingabe von einem externen Programmiergerät automatisch initiiert, wenn das mindestens eine implantierbare Lead mit der IMD verbunden ist.

6. IMD nach einem oder mehreren der Ansprüche 1 bis 5, wobei der Auswertungszeitraum etwa 2 Minuten bis etwa 1 Stunde nach Anschließen des mindestens einen Leads an die IMD beträgt.

7. IMD nach einem oder mehreren der Ansprüche 1 bis 6, ferner umfassend einen Signalgenerator (84), der konfiguriert ist, um Stimulationsimpulse über das implantierbare medizinische Lead abzugeben, wobei der Prozessor den Signalgenerator steuert, um Stimulationsimpulse in einem asynchronen Modus während des Qualifizierungstests (1) und in einem Bedarfsstimulationsmodus während einer Durchführung des Qualifizierungstests (2) abzugeben.

8. IMD nach einem oder mehreren der Ansprüche 1 bis 7, wobei, für den Qualifizierungstest (2), der Prozessor (80) konfiguriert ist zum:
Bestimmen, dass eine Schwellenanzahl von R-Zacken nicht erfasst wurde; und
Reduzieren einer Rate der Stimulationsimpulse basierend auf der Bestimmung.

9. IMD nach einem oder mehreren der Ansprüche 1 bis 6, ferner umfassend einen Signalgenerator (84), der konfiguriert ist, um Stimulationsimpulse über das implantierbare medizinische Lead abzugeben,
wobei die Vorrichtungstestsequenz ferner einen Qualifizierungstest (3), in dem der Prozessor den Signalgenerator und die Erfassungsschaltung steuert, um zu bestimmen, ob eine Stimulationsaufnahmeschwelle, PCT, ein oder mehrere Kriterien erfüllt, umfasst.

10. IMD nach Anspruch 9, wobei der Prozessor (80) die Durchführung von Qualifizierungstests (2)-(3) nach Qualifizierungstest (1) steuert.

11. IMD nach Anspruch 9 oder 10, wobei der Prozessor (80) die Erfassungsschaltung steuert, um Verletzungsstromparameter, COI, in dem EGM nach dem Qualifizierungstest (3) zu messen.

12. IMD nach Anspruch 9 oder 10, wobei der Prozessor (80) die Durchführung von Qualifizierungstests (3) an dem Ende der Vorrichtungstestsequenz steuert und die Stimulationsaufnahme durch einen Kliniker über einen Elektrokardiogrammmonitor als Angabe für Erfolg der Vorrichtungstestsequenz beobachtbar ist.

13. IMD nach einem oder mehreren der Ansprüche 1 bis 12, wobei der Prozessor (80) die IMD steuert, um, als Reaktion auf Erfolg der Vorrichtungstestsequenz, ein Bestätigungssignal zu erzeugen.

14. IMD nach Anspruch 13, ferner umfassend einen Signalgenerator (84), der konfiguriert ist, um Stimulationsimpulse über das implantierbare medizinische Lead abzugeben, wobei das Bestätigungssignal die Abgabe von Stimulationsimpulsen umfasst.

15. Leadlose implantierbare medizinische Vorrichtung, IMD, umfassend:
ein Gehäuse (730, 738), das für Implantation in einen Patienten konfiguriert ist;
eine Vielzahl von Elektroden (730, 740) auf dem Gehäuse;
die Erfassungsschaltung innerhalb des Gehäuses, wobei die Erfassungsschaltung konfiguriert ist, um ein Elektrogrammsignal, EGM-Signal, des Patienten über die Elektroden zu erfassen;
die Impedanzmessschaltung (92) innerhalb des Gehäuses, wobei die Impedanzmessschaltung konfiguriert ist, um die Impedanz über die Elektroden zu messen; und
einen Prozessor (80), der konfiguriert ist, um eine Platzierung der IMD (700) in den Patienten zu detektieren, und, als Reaktion auf die Platzierung der IMD in den Patienten, eine Vorrichtungstestsequenz zu initiieren, umfassend eine Vielzahl von Qualifizierungstests über einen Auswertungszeitraum, in dem der Prozessor:
(1) die Impedanzmessschaltung steuert, um eine Impedanz für mindestens einen elektrischen Pfad zwischen den Elektroden zu messen; und
(2) EGM-Amplituden des Patienten über einen EGM-Testzeitraum mit einer zuvor bestimmten Schwelle vergleicht.

## Revendications

1. Dispositif médical implantable, DMI, configuré pour être couplé à au moins une sonde médicale implantable (112), dans lequel le DMI (126) comprend :
un circuit de détection configuré pour détecter un signal d'électrogramme, EGM, d'un patient par l'intermédiaire d'au moins une électrode de la sonde médicale implantable ;
un circuit de mesure d'impédance (92) pour mesurer l'impédance par l'intermédiaire de la sonde médicale implantable ; et
un processeur (80) configuré, en réponse au couplage du DMI à l'au moins une sonde médicale implantable, pour initier une séquence de test de dispositif comprenant une pluralité de tests de qualification sur une période d'évaluation au cours de laquelle le processeur :
(1) commande le circuit de mesure d'impédance pour mesurer une impédance pour au moins un trajet électrique qui comporte l'au moins une électrode afin de déterminer un état de connexion du DMI à l'au moins une électrode ; et
(2) compare des amplitudes d'EGM du patient sur une période de test d'EGM à un seuil prédéterminé.

2. DMI selon la revendication 1, dans lequel le processeur (80) commande le circuit de détection pour mesurer des paramètres de courant de lésion, COI, dans l'EGM suivant le test de qualification (1).

3. DMI selon la revendication 2, dans lequel les paramètres de courant de lésion, COI, comportent un ou plusieurs parmi une amplitude maximale d'un segment ST, une amplitude du segment ST 80 millisecondes à partir d'un début du segment, une aire sous une courbe d'onde à partir d'un début de l'onde R jusqu'à la fin du segment ST, une aire sous le segment ST, une amplitude à un début du segment ST, des amplitudes médiane et quartile de l'EGM suivant le segment ST, une amplitude d'une onde R, une durée du segment ST, une durée du signal, QT, un rapport de l'amplitude de l'onde R à l'amplitude maximale du segment ST, ou un rapport de l'amplitude de l'onde R à l'amplitude du segment ST 80 ms à partir du début.

4. DMI selon la revendication 2 ou 3, dans lequel le processeur commande le circuit de détection pour mesurer les paramètres de COI en parallèle avec le test de qualification (2).

5. DMI selon l'une quelconque ou plusieurs des revendications 1 à 4, dans lequel le processeur (80) initie automatiquement la séquence de test de dispositif, sans intervention d'un programmateur externe, lorsque l'au moins une sonde implantable est connectée au DMI.

6. DMI selon l'une quelconque ou plusieurs des revendications 1 à 5, dans lequel la période d'évaluation est d'environ 2 minutes à environ 1 heure suivant la fixation de l'au moins une sonde au DMI.

7. DMI selon l'une quelconque ou plusieurs des revendications 1 à 6, comprenant en outre un générateur de signaux (84) configuré pour administrer des impulsions de stimulation par l'intermédiaire de la sonde médicale implantable, dans lequel le processeur commande le générateur de signaux pour administrer des impulsions de stimulation dans un mode asynchrone pendant le test de qualification (1) et un mode de stimulation à la demande pendant la réalisation du test de qualification (2).

8. DMI selon l'une quelconque ou plusieurs des revendications 1 à 7, dans lequel, pour le test de qualification (2), le processeur (80) est configuré pour :
déterminer qu'un nombre seuil d'ondes R n'a pas été détecté ; et
réduire une fréquence des impulsions de stimulation en fonction de la détermination.

9. DMI selon l'une quelconque ou plusieurs des revendications 1 à 6, comprenant en outre un générateur de signaux
(84) configuré pour administrer des impulsions de stimulation par l'intermédiaire de la sonde médicale implantable, dans lequel la séquence de test de dispositif comprend en outre un test de qualification (3) dans lequel le processeur commande le générateur de signaux et le circuit de détection pour déterminer si un seuil de capture de stimulation, PCT, satisfait à un ou plusieurs critères.

10. DMI selon la revendication 9, dans lequel le processeur (80) commande la réalisation des tests de qualification (2)-(3) suivant le test de qualification (1).

11. DMI selon la revendication 9 ou 10, dans lequel le processeur (80) commande le circuit de détection pour mesurer des paramètres de courant de lésion, COI, dans l'EGM après le test de qualification (3).

12. DMI selon la revendication 9 ou 10, dans lequel le processeur (80) commande la réalisation du test de qualification (3) à la fin de la séquence de test de dispositif, et la capture de stimulation peut être observée par un clinicien par l'intermédiaire d'un moniteur d'électrocardiogramme en tant qu'indication du succès de la séquence de test de dispositif.

13. DMI selon l'une quelconque ou plusieurs des revendications 1 à 12, dans lequel le processeur (80) commande le DMI pour générer un signal de confirmation en réponse au succès de la séquence de test de dispositif.

14. DMI selon la revendication 13, comprenant en outre un générateur de signaux (84) configuré pour administrer des impulsions de stimulation par l'intermédiaire de la sonde médicale implantable, dans lequel le signal de confirmation comprend l'administration d'impulsions de stimulation.

15. Dispositif médical implantable sans sonde, DMI, comprenant :
un logement (730, 738) configuré pour une implantation à l'intérieur d'un patient ;
une pluralité d'électrodes (730, 740) sur le logement ;
un circuit de détection à l'intérieur du logement, le circuit de détection étant configuré pour détecter un signal d'électrogramme, EGM, du patient par l'intermédiaire des électrodes ;
un circuit de mesure d'impédance (92) à l'intérieur du logement, le circuit de mesure d'impédance étant configuré pour mesurer l'impédance par l'intermédiaire des électrodes ; et
un processeur (80) configuré pour détecter la mise en place du DMI (700) chez le patient et, en réponse à la mise en place du DMI chez le patient, pour initier une séquence de test de dispositif comprenant une pluralité de tests de qualification sur une période d'évaluation au cours de laquelle le processeur :
(1) commande le circuit de mesure d'impédance pour mesurer une impédance pour au moins un trajet électrique entre les électrodes ; et
(2) compare des amplitudes d'EGM du patient sur une période de test d'EGM à un seuil prédéterminé.
